# EUROPEAN PATENT APPLICATION

(11) **EP 4 219 473 A1**
(43) Date of publication of application: **02.08.2023**
(21) Application number: 21886904.8
(22) Date of filing: 29.10.2021
(51) Int. Cl.: C07D 403/06, A61K 31/5377, A61P 3/04, A61P 3/10, A61P 29/00, A61P 15/10

(54) **CRYSTAL TYPE II OF MALANOCORTIN RECEPTOR AGONIST COMPOUND AND METHOD FOR PREPARING SAME**

(30) Priority: 29.10.2020 KR 20200142397
(71) Applicant: Lg Chem, Ltd., Seoul 07336 (KR)
(72) Inventor: HAM, Jin Ok, Daejeon 34122 (KR); LEE, Ho Yeon, Daejeon 34122 (KR); KIM, Ji Yoon, Daejeon 34122 (KR); KIM, Sung Won, Daejeon 34122 (KR); CHUN, Seul Ah, Daejeon 34122 (KR); LEE, Sang Dae, Daejeon 34122 (KR); PARK, Jong Won, Daejeon 34122 (KR)
(74) Representative: Hoffmann Eitle
(86) International application number: PCT/KR2021/015470
(87) International publication number: WO 2022/092910

(57) **Abstract**

The present invention relates to a crystal type II of a compound represented by chemical formula 1, a method for preparing same, and a pharmaceutical composition including same. The crystal type II of the compound represented by chemical formula 1 of the present invention can be characterized by an XRD pattern, a DSC profile, and/or a TGA profile.

## Description

### TECHNICAL FIELD

### Cross-reference to Related Applications

The present application claims the benefit of priority based on Korean Patent Application No. 10-2020-0142397, filed on 29 October 2020, the entire disclosure of which is incorporated as part of the specification.

### Technical Field

The present invention relates to a crystalline form II of a novel compound exhibiting an excellent agonistic activity for a melanocortin receptor, a method for preparing the same, and a pharmaceutical composition comprising the same.

### BACKGROUND ART

Leptin protein is a hormone secreted by adipocytes, and its secretion amount increases with an increase in body fat content. It regulates functions of various neuropeptides produced from hypothalamus, thereby regulating various in vivo functions, including appetite, body fat content, and energy metabolism (Schwartz, et al., Nature 404, 661-671 (2000)). The leptin protein signal transduction for controlling appetite and body weight is made through the regulation of many factors downstream, the most representative of which are melanocortin, agouti-related peptide (AgRP), and neuropeptide Y (NPY) hormones.

When the concentration of leptin in the blood increases as a result of excess calories in vivo, the secretion of proopiomelanocortin (POMC) protein hormone from the pituitary gland increases and the production of AgRP and NPY decreases. A small peptide hormone, alpha-melanocyte-stimulating hormone (MSH), is produced from POMC neurons. The hormone is an agonist for melanocortin-4 receptors (MC4R) of second-order neurons and ultimately induces appetite decrease. Meanwhile, when the concentration of leptin decreases as a result of calorie deficiency, the expression of AgRP, an MC4R antagonist, increases, and the expression of NPY also increases, which ultimately promotes appetite. That is, according to the change of leptin, the alpha-MSH hormone and the AgRP hormone act as agonists and antagonists for MC4R and thus are involved in appetite control.

The Alpha-MSH hormone binds to three MCR subtypes in addition to MC4R to induce various physiological reactions. Five MCR subtypes have been identified so far. Among them, MC1R is expressed mainly in skin cells and is involved in regulating melanin pigmentation (skin pigmentation). MC2R is expressed mainly in the adrenal gland and is known to be involved in the production of glucocorticoid hormones. Its ligand is only adrenocorticotropic hormone (ACTH) derived from POMC. MC3R and MC4R, which are expressed mainly in the central nervous system, are involved in regulating appetite, energy metabolism, and body fat storage efficiency, and MC5R expressed in various tissues is known to regulate exocrine function (Wikberg, et al., Pharm Res 42 (5) 393-420 (2000)). In particular, activation of the MC4R receptor induce appetite decrease and energy metabolism increase and thus has an effect of efficiently reducing body weight. Therefore, it has been proven to be a major action point in the development of anti-obesity drugs (Review: Wikberg, Eur. J. Pharmacol 375, 295-310 (1999)); Wikberg, et al., Pharm Res 42 (5) 393-420 (2000); Douglas et al., Eur J Pharm 450, 93-109 (2002); O'Rahilly et al., Nature Med 10, 351-352 (2004)).

The role of MC4R in the control of appetite and body weight was primarily demonstrated through an experiment in an animal model of abnormal expression of the agouti protein (agouti mouse). In the case of the Agouti mouse, it was found that due to genetic mutation, the agouti protein was expressed at a high concentration in the central nervous system and acted as an antagonist of MC4R in the hypothalamus to cause obesity (Yen, TT et al., FASEB J. 8, 479-488 (1994); Lu D., et al. Nature 371, 799-802 (1994)). Subsequent research results showed that the agouti-related peptides (AgRP) similar to the actual agouti protein were expressed in hypothalamic nerves, and these are also known to be antagonists for MC4R and be involved in controlling appetite (Shutter, et al., Genes Dev., 11, 593-602 (1997); Ollman, et al. Science 278, 135-138 (1997)).

Intracerebral administration of alpha-MSH, which is an in vivo MC4R agonist, to animals leads to the effect of reducing appetite. When treating the animals with SHU9119 (peptide) or HS014 (peptide), which are MC4R antagonists, it was observed that appetite increased again (Kask et al., Biochem. Biophys. Res. Comm. 245, 90-93 (1998)). In addition, in animal studies using Melanotan II (MTII, Ac-Nle-c[Asp-His-DPhe-Arg-Trp-Lys]-NH2) and the similar agonist thereof, HP228, after intracerebral, intraperitoneal, or subcutaneous administration, efficiencies of inhibiting appetite, reducing body weight, increasing energy metabolism, etc. were found. (Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). On the contrary, administration of the representative SHU9119 to animals showed significant and sustained feed intake and weight gain, providing pharmacological evidence that MCR agonists could be anti-obesity agents. The effect of reducing appetite, which is clearly exhibited upon administration of MTII, was not observed in MC4R knock-out (KO) mice. This experimental result proves again that the appetite-reducing effect is achieved mainly through the activation of MC4R (Marsh, et al., Nat Genet 21, 119-122 (1999)).

Anorectic agents acting on the central nervous system were the main types of antiobestic drugs developed so far. Among them, most were drugs that modulate the action of neurotransmitters. Examples include noradrenalin agents (phentermine and mazindol), serotonergic agents, fluoxetine and sibutramine, and the like. However, the neurotransmitter modulators have a wide range of effects on various physiological actions in addition to appetite suppression, through numerous subtype receptors. Accordingly, the modulators lack selectivity for each subtype, and thus have a major disadvantage in that they are accompanied by various side effects when administered for a long period.

Meanwhile, melanocortin agonists are neuropeptides, not neurotransmitters. Given that in MC4R gene KO mice, all functions other than energy metabolism are normal, they have an advantage as an action point in that they can induce only weight loss through appetite suppression without affecting other physiological functions. In particular, the receptor is a G-protein coupled receptor (GPCR) that belongs to the most successful category of new drug action points developed so far. Thus, the action point greatly differs from existing action points in that it is relatively easy to secure selectivity for subtype receptors.

As an example of utilizing a melanocortin receptor as an action point, international publication nos. WO 2008/007930 and WO 2010/056022 disclose compounds as agonists of the melanocortin receptor.

In addition, the inventors of the present invention have conducted extensive studies and invented a novel compound of the following formula 1 having an excellent agonistic activity selective for a melanocortin receptor, in particular, melanocortin-4 receptor (MC4R), and a method for preparing the same (application no. KR 10-2019-0141649 (filed on 7 November 2019)): 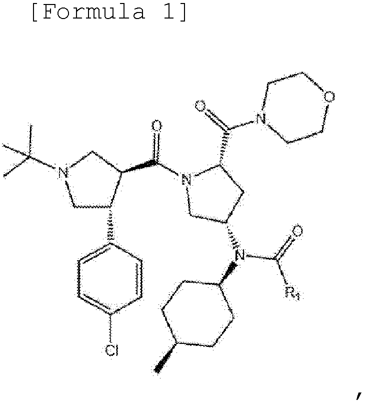 wherein R₁ is C₂-C₅ alkyl.

Meanwhile, the crystal structure of a pharmaceutically active ingredient often affects the chemical stability of the drug. Different crystallization conditions and storage conditions can lead to changes in the crystal structure of the compound, and sometimes the accompanying production of other forms of the crystalline form. In general, an amorphous drug product does not have a regular crystal structure, and often has other defects such as poor product stability, smaller particle size, difficult filtration, easy agglomeration, and poor flowability. Thus, it is necessary to improve various physical properties of the product. As such, it is necessary to study crystal structures having high purity and good chemical stability for a single compound.

### [Prior art documents]

### [Patent Documents]

(Patent Document 1) International Patent Application Publication No. WO 2008/007930
(Patent Document 2) International Patent Application Publication No. WO 2010/056022

### DISCLOSURE OF THE INVENTION

### TECHNICAL PROBLEM

An aspect of the present invention provides a stable crystalline form of a novel compound having an excellent agonistic activity, which is selective for a melanocortin receptor, in particular, melanocortin-4 receptor (MC4R), and a method for preparing the same.

Another aspect of the present invention provides a pharmaceutical composition comprising the stable crystalline form of the novel compound.

### TECHNICAL SOLUTION

According to an aspect of the present invention, there is provided a crystalline form II of a compound of the following formula 1, a pharmaceutically acceptable salt, or a solvate thereof,

wherein the X-ray powder diffraction pattern has 3 or more, 5 or more, 7 or more, 9 or more, or 10 or more characteristic peaks selected from among peaks with the following diffraction angles (2θ values) of: 7.77±0.2°, 9.8210.2°, 10.5010.2°, 11.3710.2°, 12.3610.2°, 15.1710.2°, 15.4610.2°, 15.8810.2°, 16.7510.2°, 17.5910.2°, 17.9310.2°, 18.3310.2°, 19.6410.2°, 20.1910.2°, 21.1910.2°, 21.7110.2°, 23.2910.2°, 23.5810.2°, 24.4210.2°, 25.07±0.2°, 25.6310.2°, 26.3110.2°, 27.1710.2°, 27.5210.2°, and 28.96±0.2°, 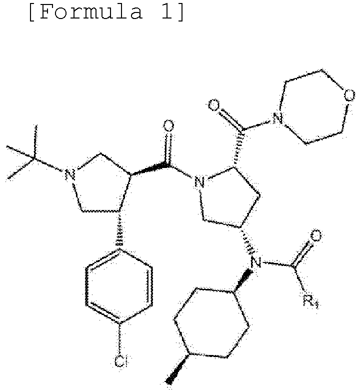 wherein R₁ is C₂-C₅ alkyl.

Since the compound of formula 1 can have an asymmetric carbon center and an asymmetric axis or an asymmetric plane, it can exist as cis or trans isomers, R or S isomers, racemates, diastereomer mixtures, and individual diastereomers, all of which are within the scope of the compound of formula 1.

In the present specification, unless otherwise specified for convenience, the compound of formula 1 is used to include all of the compound of formula 1, a pharmaceutically acceptable salt, an isomer, and a solvate thereof.

In one embodiment according to the present invention, in formula 1, R₁ is C₂ to C₅ alkyl. In another embodiment according to the present invention, in formula 1, R₁ is a straight-chain or branched C₂ to C₅ alkyl, for example, ethyl, n-propyl, iso-propyl, n-butyl, iso-butyl, sec-butyl, or tert-butyl.

In another embodiment according to the present invention, in formula 1, R₁ is C₂ or C₃ alkyl. In another embodiment according to the present invention, in formula 1, R₁ is a straight-chain or branched C₂ or C₃ alkyl, for example, ethyl, n-propyl, or iso-propyl.

In one embodiment according to the present invention, the pharmaceutically acceptable salt includes, but are not limited to, acid-addition salts which are formed from inorganic acids, such as hydrochloric acid, sulfuric acid, nitric acid, phosphoric acid, hydrobromic acid, and hydroiodic acid; organic carbonic acids, such as tartaric acid, formic acid, citric acid, acetic acid, trichloroacetic acid, trifluoroacetic acid, gluconic acid, benzoic acid, lactic acid, fumaric acid, and maleic acid; or sulfonic acids, such as methanesulfonic acid, benzenesulfonic acid, p-toluenesulfonic acid, or naphthalene-sulfonic acid.

In one embodiment according to the present invention, the solvate may include a hydrate; and a solvate with an organic solvent, such as methanol, ethanol, 2-propanol, 1,2-propanediol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, and mixtures thereof.

In one embodiment according to the present invention, the crystalline form II may be a crystalline form of the pharmaceutically acceptable salt of the compound of formula 1.

The pharmaceutically acceptable salt of the compound of formula 1 may be a hydrochloride compound of the following formula 2: 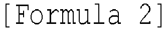 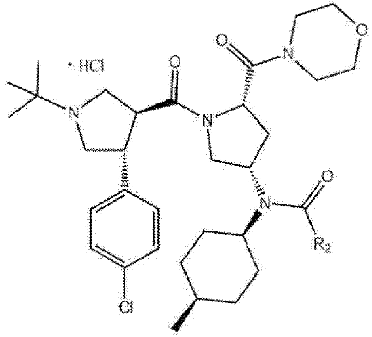 wherein R₂ is C₂-C₅ alkyl.

In another embodiment according to the present invention, the pharmaceutically acceptable salt of the compound of formula 1 may be *N*-((3*S*,5*S*)-1-((3*S*,4*R*)-1-(*tert-*butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide hydrochloride of the following formula 3.

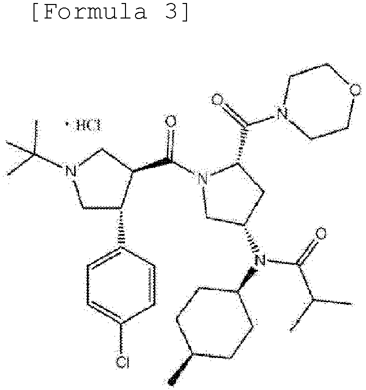

In another embodiment according to the present invention, the crystalline form II may be a crystalline form of the solvate, specifically the hydrate of the pharmaceutically acceptable salt of the compound of formula 1.

More specifically, the crystalline form II may be a crystalline form of the hydrate of the hydrochloride salt of the compound of formula 1.

In one embodiment according to the present invention, the crystalline form II may be a crystalline form of the compound of the following formula 4.

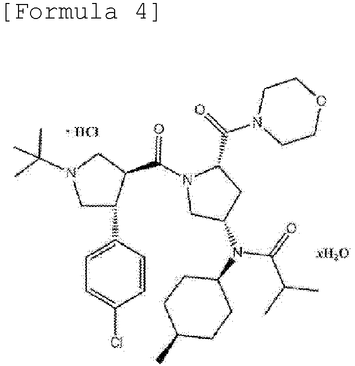

The crystalline form II according to the present invention shows 3 or more, 5 or more, 7 or more, 9 or more, or 10 or more characteristic peaks selected from among peaks with 2θ values of: 7.77±0.2°, 9.8210.2°, 10.5010.2°, 11.3710.2°, 12.3610.2°, 15.1710.2°, 15.4610.2°, 15.8810.2°, 16.7510.2°, 17.5910.2°, 17.9310.2°, 18.3310.2°, 19.6410.2°, 20.1910.2°, 21.1910.2°, 21.7110.2°, 23.2910.2°, 23.5810.2°, 24.4210.2°, 25.07±0.2°, 25.6310.2°, 26.3110.2°, 27.1710.2°, 27.5210.2°, and 28.96±0.2°, as analyzed by X-ray powder diffraction (XRD).

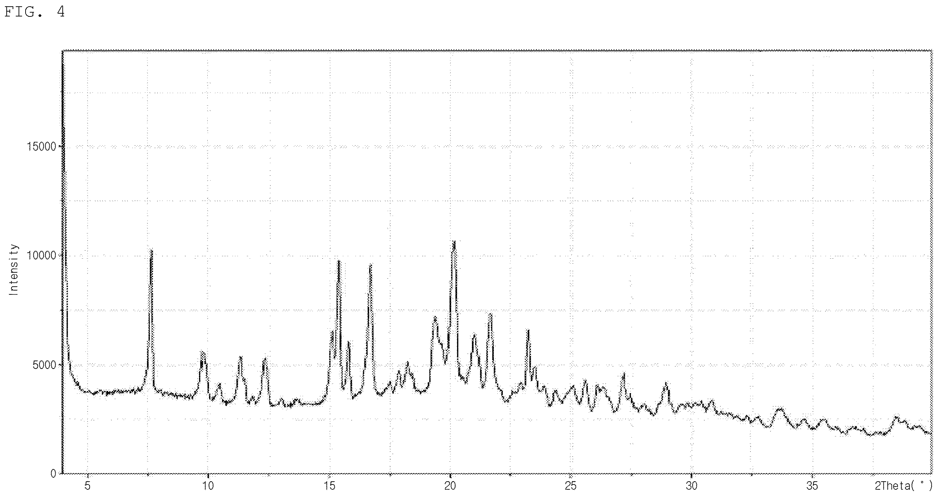

In one embodiment according to the present invention, the crystalline form II may have the XRD pattern shown in FIG. 4.

In the differential scanning calorimetry (DSC) profile of the crystalline form II according to the present invention, two endothermic peaks appear at 30 to 200°C, and an endothermic peak due to decomposition appears at 220°C or higher.

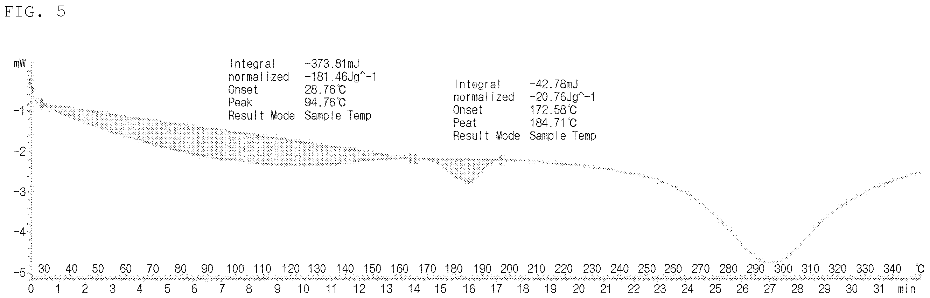

In one embodiment according to the present invention, the crystalline form II may have the DSC profile shown in FIG. 5.

The crystalline form II according to the present invention, in the thermogravimetric analysis (TGA) profile, may have 15% or less, for example, 1% to 15%, 1% to 10%, 5% to 10%, or 6% of weight loss when heated to a temperature of 170°C or less.

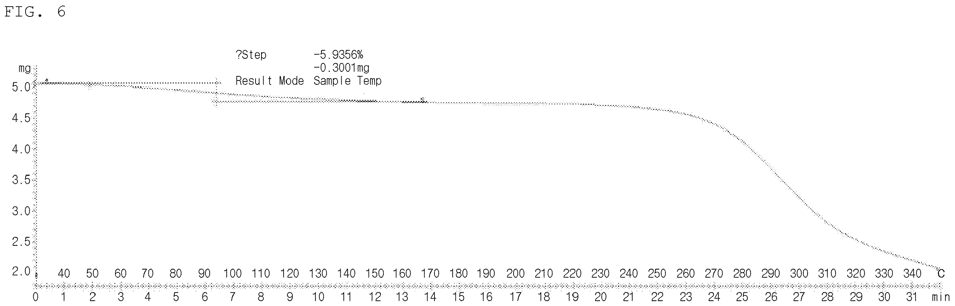

In one embodiment according to the present invention, the crystalline form II may have the TGA profile shown in FIG. 6.

The stability test result (HPLC) showed that the crystalline form II according to the present invention exhibited chemical stability for 4 weeks under the accelerated condition (40°C, 75% RH) and the harsh condition (80°C). Thus, it can be seen that the crystalline form II according to the present invention is stable against heat and humidity.

In the present specification,
X-ray diffraction (XRD) analysis shows the results performed using PANalytical X' Pert Pro MPD system (Malvern Panalytical Ltd.).

Differential scanning calorimetry (DSC) analysis shows the results performed using DSC1 (Mettler-Toledo AG).

Thermogravimetric analysis (TGA) shows the results performed using TGA/DSC 1 (Mettler-Toledo AG).

Stability analysis shows the results performed using HPLC (Agilent Technologies, Inc.).

The crystalline form II may have higher purity than a crude compound of formula 1, an amorphous compound of formula 1, or other crystalline forms of the compound of formula 1, and may be physically and chemically more stable.

Moreover, the agonistic ability for the melanocortin-4 receptor and preventive or therapeutic effects on diseases, such as obesity, diabetes, inflammation, erectile dysfunction, or the like, of the crystalline form II of the compound of formula 1, can be more excellent than those of known melanocortin-4 receptor agonists. However, the effects of the present invention are not limited thereto.

In another aspect, the present invention provides a method for preparing the crystalline form II, comprising the steps of: preparing a mixed solution by dissolving the compound of formula 1 in a crystallization solvent; and obtaining crystals from the mixed solution.

First, the compound represented by formula 1 is dissolved in a crystallization solvent.

The compound of formula 1 for preparing the crystalline form II may be a compound of formula 1, a salt thereof, an isomer thereof, or a solvate thereof.

The compound of formula 1 may be obtained by the preparation method described in the specification of application no. KR 10-2019-0141649 (filed on 7 November 2019) .

The crystallization solvent may be used without particular limitation as long as it is a suitable solvent for crystallization of compounds. In one embodiment, the crystallization solvent includes a mixture of water and a polar aprotic organic solvent.

The polar aprotic organic solvent may include ethyl acetate, methyl isobutyl ketone, dimethyl sulfoxide, tetrahydrofuran, acetone, dimethylformamide, acetonitrile, or a mixture thereof.

In one embodiment according to the present invention, the polar aprotic organic solvent may include ethyl acetate.

In one embodiment according to the present invention, the crystallization solvent may be a mixed solvent in which water and the polar aprotic organic solvent are mixed in a volume ratio of 20:1 to 1:20, specifically 15:1 to 1:15, 10:1 to 1:10, 1:1 to 1:10, 1:5 to 1:18, 1:7 to 1:16, 1:7.5 to 1:15, 1:8 to 1:13, 1:8.5 to 1:12, 1:9 to 1:11, 1:9.5 to 1:10.5, or 1:10.

For 1 g of the compound of formula 1, 0.1 to 5 mL, 0.3 to 3 mL, 0.5 to 1.5 mL, 0.6 to 1 mL, 0.65 to 0.9 mL, 0.65 to 0.7 mL, or 0.66 mL of the crystallization solvent may be used.

Dissolution of the compound of formula 1 in the crystallization solvent may be carried out without or with stirring at room temperature, for example, 20 to 30°C, specifically 23 to 28°C, or 25°C.

In one embodiment according to the present invention, a mixed solution in which the compound of formula 1 has been dissolved at room temperature may be obtained by using 0.6 mL of EtOAc and 0.06 mL of distilled water with respect to 1 g of the compound of formula 1.

Next, the method includes the step of obtaining crystals from the mixed solution in which the compound of formula 1 has been dissolved. The crystals may be obtained, for example, by cooling the solution, by adding an acid dropwise to the solution to form a precipitate, by evaporating the solvent, by adding an antisolvent for supersaturation, or by using methods, such as slurry conversion, or the like.

In addition, the crystallization step may include stirring the mixed solution. Herein, the method may further include a step for adding a non-polar organic solvent to the mixed solution before, after, or simultaneously with stirring the mixed solution.

The stirring may be performed by known means, and the stirring time is, for example, but not limited to, 10 hours to 24 hours (inclusive), specifically hours, 10 hours to 24 hours, 12 hours to 20 hours, 15 hours to 19 hours, 16 hours to 19 hours, or 18 hours.

In one embodiment according to the present invention, the precipitate formed by stirring while adding the non-polar organic solvent dropwise to the mixed solution may be filtered and washed to obtain crystals.

The yield or production stability of the obtained crystalline form II may be improved by adding the non-polar organic solvent to increase the production rate of crystallized particles, but the present invention is not limited thereto.

The non-polar organic solvent may be used without particular limitation as long as it is an organic solvent having non-polar properties, but, for example, hexane, heptane, cyclohexane, carbon tetrachloride, benzene, chloroform, and the like, may be used.

In one embodiment according to the present invention, the method may include a step for adding heptane to the solution during crystallization from the mixed solution.

The crystalline form II as obtained above may have higher purity than a crude compound of formula 1, an amorphous compound of formula 1, or any other crystalline forms of formula 1, and may be physically and chemically more stable. However, the effects of the present invention are not limited thereto.

In another aspect, the present invention provides a pharmaceutical composition comprising: (i) the crystalline form II; and (ii) a pharmaceutically acceptable carrier.

The crystalline form II according to the present invention exhibits excellent agonistic actions on melanocortin receptors, in particular, melanocortin-4 receptors (MC4R). Thus, the present invention can provide a pharmaceutical composition for agonizing melanocortin receptors, the composition containing the above-described crystalline form II as an active ingredient. Specifically, the pharmaceutical composition may be a composition for agonizing the function of the melanocortin-4 receptor.

In addition, since the pharmaceutical composition can exhibit excellent effects of preventing or treating obesity, diabetes, inflammation, and erectile dysfunction, it may be a composition for preventing or treating obesity, diabetes, inflammation, or erectile dysfunction. However, the use of the present invention is not limited the diseases.

As used herein, "carrier" refers to a compound that facilitates the introduction of compounds into a cell or tissue.

When the crystalline form II of the present invention is administered for clinical purposes, the total daily dose to be administered to a host in a single dose or in divided doses may be preferably in the range of 0.01 to 10 mg/kg body weight. However, the specific dose level for an individual patient may vary depending on the specific compound to be used, the patient's weight, sex, health status, diet, administration time of the drug, administration method, excretion rate, drug combination, the severity of the disease, or the like.

The crystalline form II of the present invention may be administered by any route as desired. For example, the amorphous compound of the present invention may be administered by injection or orally.

The pharmaceutical composition of the present invention may be in various oral dosage forms, such as tablets, pills, powders, capsules, granules, syrups, or emulsions, or parenteral dosage forms, such as injection preparations for intramuscular, intravenous, or subcutaneous administration.

Preparations for injection may be prepared according to known techniques using suitable dispersing agents, wetting agents, suspending agents, or excipients.

Excipients that can be used in the pharmaceutical preparation of the present invention include, but are not limited to, sweeteners, binders, solubilizers, solubilizing agents, wetting agents, emulsifiers, isotonic agents, adsorbents, disintegrants, antioxidants, preservatives, lubricants, fillers, fragrances, etc. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinyl pyrrolidone, sodium chloride, calcium chloride, orange essence, strawberry essence, vanilla flavor, etc. may be used.

When the pharmaceutical composition of the present invention is in an oral dosage form, examples of the carrier to be used may include, but are not limited to, cellulose, calcium silicate, corn starch, lactose, sucrose, dextrose, calcium phosphate, stearic acid, magnesium stearate, calcium stearate, gelatin, talc, etc.

When the pharmaceutical composition of the present invention is in an injectable preparation form, examples of the carrier may include, but are not limited to, water, saline, aqueous glucose solution, an aqueous sugar-like solution, alcohols, glycol, ethers, oils, fatty acids, fatty acid esters, glycerides, etc.

In another aspect, there is provided a crystalline form II as described above for use in agonizing the functions of melanocortin receptors, in particular, melanocortin-4 receptors (MC4R).

In one embodiment, there is provided a crystalline form II as described above for use in treating or preventing obesity, diabetes, inflammation, or erectile dysfunction.

In another aspect, there is provided a method for agonizing the function of melanocortin receptors, in particular, melanocortin-4 receptors (MC4R), the method comprising a step for administering to a subject the above-described crystalline form II.

In another aspect, there is provided a method for treating obesity, diabetes, inflammation, or erectile dysfunction, the method comprising a step for administering to a subject the above-described crystalline form II.

### ADVANTAGEOUS EFFECTS

The crystalline form II according to the present invention exhibits excellent agonistic action on melanocortin receptors, in particular, melanocortin-4 receptors (MC4R), and thus can be usefully used for preventing or treating obesity, diabetes, inflammation, and erectile dysfunction.

The crystalline form II according to the present invention exhibits an on-target effect on melanocortin-4 receptors, thereby exhibiting weight loss and diet reduction effects, without affecting anxiety and depression. In addition, it can be administered without any safety issues, such as side effects of human ether-a-go-go related gene (hERG) inhibition or mutagenesis.

In addition, the crystalline form II, according to the present invention, has purity, yield, physical and chemical stability, which are more excellent than the crude compound of formula 1, the amorphous compound of formula 1, or any other crystalline forms of formula 1.

Specifically, the crystalline form II may have superior solubility, storage stability, and production stability to the compound of formula 1, the amorphous compound of formula 1, or any other crystalline forms of formula 1.

### BRIEF DESCRIPTION OF THE DRAWINGS

FIG. 1 is the graph of the XRD result of Preparation Example 4.
FIG. 2 is the graph of the DSC result of Preparation Example 4.
FIG. 3 is the graph of the TGA result of Preparation Example 4.
FIG. 4 is the graph of the XRD result of Example 1.
FIG. 5 is the graph of the DSC result of Example 1.
FIG. 6 is the graph of the TGA result of Example 1.

### MODE FOR CARRYING OUT THE INVENTION

Hereinafter, the present invention will be described in more detail through Preparation Examples and Examples. However, these Examples are merely illustrative of the present invention, and the scope of the present invention is not limited thereto.

### Preparation Example 1: Preparation of methyl (2S,4S)-4-(N-((1s,4P)-4-methylcyclohexyl)isobutyramido)pyrrolidine-2-carboxylate hydrochloride

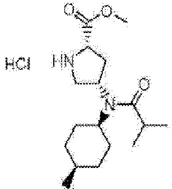

The title compound was obtained through the following steps A, B, C, D, and E.

### Step A: Preparation of 1-(tert-butyl) 2-methyl (2S,4S)-4-azidopyrrolidine-1,2-dicarboxylate

1-(tert-butyl) 2-methyl (2S,4R)-4-((methylsulfonyl)oxy)pyrrolidine-1,2-dicarboxylate (48.5 g, 150 mmol) was dissolved in N,N'-dimethylformamide (250 ml) under nitrogen, and sodium azide (19.5 g, 300 ml) was added. After stirring at 80°C for 16 hours, the reaction solvent was concentrated under reduced pressure, water was added, and extraction was performed twice with ethyl acetate. The organic layer was washed with an aqueous sodium chloride solution and water, dried over anhydrous magnesium sulfate, and filtered. The filtrate was concentrated under reduced pressure to obtain crude 1-(tert-butyl) 2-methyl (2S,4S)-4-azidopyrrolidine-1,2-dicarboxylate (39.59 g, 98%), which was used in the next step without purification.
MS [M+H] = 271 (M+1)
¹H NMR (400 MHz, CD3OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

1-(tert-butyl) 2-methyl (2S,4S)-4-azidopyrrolidine-1,2-dicarboxylate (24.59 g, 91.0 mmol) obtained in step A above was dissolved in tetrahydrofuran (180 ml), and 1 M trimethylphosphine tetrahydro solution (109.2 ml, 109.2 mmol) was slowly added at 0°C. After stirring at the same temperature for 1 hour, the mixture was stirred at room temperature for 3 hours. After the reaction solvent was concentrated under reduced pressure, dichloromethane (100 ml) and water (150 ml) were added, and the mixture was stirred for about 30 minutes. The layers were separated and were extracted once more with dichloromethane, and the organic layer was dried over anhydrous magnesium sulfate and was filtered. The filtrate was concentrated under reduced pressure to obtain crude 1-(tert-butyl) 2-methyl (2S,4S)-4-aminopyrrolidine-1,2-dicarboxylate (20.62 g, 93%), which was used in the next step without purification.
MS [M+H] = 245 (M+1)
¹H NMR (400 MHz, CD3OD) δ 4.27 (m, 1H), 3.77 (s, 1.8H), 3.76 (s,1.2H), 3.75-3.67 (m, 1H), 3.50-3.42 (m, 1H), 3.22-3.17 (m, 1H), 2.58-2.47 (m,1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

1-(tert-butyl) 2-methyl (2S,4S)-4-aminopyrrolidine-1,2-dicarboxylate (20.62 g, 84.4 mmol) obtained in step B above was dissolved in dichloroethane (150 ml), and 4-methylcyclohexanone (9.5 ml, 101.3 mmol) was added. Sodium triacetoxyborohydride (26.8 g, 126.6 mmol) was added at 0°C, and the mixture was stirred at room temperature for 16 hours. The reaction solvent was concentrated under reduced pressure, water was added, and extraction was performed twice with ethyl acetate. The organic layer was washed with an aqueous sodium chloride solution, dried over anhydrous magnesium sulfate, and filtered. The filtrate was concentrated under reduced pressure and purified by column chromatography to obtain 1-(tert-butyl) 2-methyl (2S,4S)-4-(((1s,4R)-4-methylcyclohexyl)amino)pyrrolidine-1,2-dicarboxylate (22.9 g, 80%) .
MS [M+H] = 341 (M+1)
¹H NMR (400 MHz, CD3OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60(br, 1H), 2.58-2.46 (m, 1H), 1.87-1.77 (m, 1H), 1.73-1.63 (m, 1H), 1.62-1.35(m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido)pyrrolidine-1,2-dicarboxylate

1-(tert-butyl) 2-methyl (2S,4S)-4-(((1s,4R)-4-methylcyclohexyl)amino)pyrrolidine-1,2-dicarboxylate obtained in step C above (37.29 g, 109.5 mmol) was dissolved in dichloromethane (500 ml), triethyl amine (61.1 ml, 438.1 mmol) was added, and then isobutyryl chloride (11.7 ml, 219 mmol) was slowly added at 0°C. After stirring at room temperature for 16 hours, the reaction solvent was concentrated under reduced pressure, an aqueous sodium hydrogen carbonate solution was added, and extraction was performed twice with ethyl acetate. The organic layer was washed with an aqueous sodium chloride solution and water, dried over anhydrous magnesium sulfate, and filtered. The filtrate was concentrated under reduced pressure, and purified by column chromatography to obtain 1-(tert-butyl) 2-methyl (2S,4S)-4-(N-((1s,4R)-4-methylcyclohexyl)isobutyramido)pyrrolidine-1,2-dicarboxylate (38.79 g, 86%).
MS [M+H] = 411 (M+1)
¹H NMR (400 MHz, CD3OD) δ 4.27 (m, 1H), 3.76 (s, 1.8H), 3.75 (s, 1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of methyl (2S,4S)-4-(N-((1s,4R)-4-methylcyclohexyl)isobutyramido)pyrrolidine-2-carboxylate hydrochloride

1-(tert-butyl) 2-methyl (2S,4S)-4-(N-((1s,4R)-4-methylcyclohexyl)isobutyramido)pyrrolidine-1,2-dicarboxylate (34.0 g, 82.8 mmol) obtained in step D above was dissolved in dichloromethane (200 ml), and a solution of 4 N hydrochloric acid in 1,4-dioxane solution (82.8 ml, 331.3 mmol) was added at 0°C. After stirring at room temperature for 6 hours, the reaction solvent was concentrated under reduced pressure to obtain crude (28.7 g, 99%), which was used in the next step without purification.
MS[M+H] = 311 (M+1)

### Preparation Example 2: Preparation of (3S,4R)-1-(tert-butyl)-4-(4-chlorophenyl)pyrrolidine-3-carboxylic acid

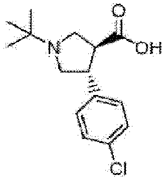

The title compound was obtained according to the method described in international patent publication no. WO 2004/092126.
MS[ M+H] = 282 (M+1)
¹H NMR (400 MHz, CD3OD) δ 7.43-7.33 (m, 4H), 3.90-3.69 (m, 3H), 3.59 (dd, J = 11.2, 10.0 Hz, 1H), 3.29 (dd, J = 11.2, 11.2 Hz, 1H), 3.18-3.09 (m, 1H), 1.44 (s, 9H)

### Preparation Example 3: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

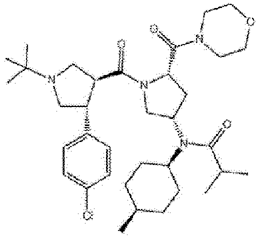

The title compound was obtained through the following steps A, B, and C.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-4-(N-((1s,4R)-4-methylcyclohexyl)isobutyramido)pyrrolidine-2-carboxylate hydrochloride (28.7 g, 82.73 mmol) obtained in Preparation Example 1, (3S,4R)-1-(tert-butyl)-4-(4-chlorophenyl)pyrrolidine-3-carboxylic acid (24.5 g, 86.87 mmol) obtained in Preparation Example 2, 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in N,N'-dimethylformamide (400 ml), and N,N'-diisopropylethylamine (72.0 ml, 413.66 mmol) was added slowly. After stirring at room temperature for 16 hours, the reaction solvent was concentrated under reduced pressure, 0.5 N sodium hydroxide aqueous solution was added, and extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (39.4 g, 68.62 mmol) obtained in step A above was dissolved in methanol (450 ml), and then, 6 N sodium hydroxide aqueous solution (57.2 ml, 343.09 mmol) was added. After stirring at room temperature for 16 hours, and adjusting the pH to about 5 with 6 N aqueous hydrochloric acid solution, the reaction solution was concentrated under reduced pressure. After dissolving the concentrate in dichloromethane, the insoluble solid was filtered through a paper filter. The filtrate was concentrated under reduced pressure to obtain the crude title compound (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2S,4S)-1-((3S,4R)-1-(tert-butyl)-4-(4-chlorophenyl)pyrrolidine-3-carbonyl)-4-(N -((1s,4R)-4-methylcyclohexyl)isobutyramido)pyrrolidine-2-carboxylic acid (38.4 g, 68.60 mmol) obtained in step B above, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N,N'-dimethylformamide (200ml), and then morpholine (5.9 ml, 68.80 mmol) and N,N'-diisopropylethylamine (59.7 ml, 343.02 mmol) were sequentially and slowly added. After stirring at room temperature for 16 hours, the reaction solution was concentrated under reduced pressure, 0.5 N sodium hydroxide aqueous solution was added, and extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (37.05 g, 86%).
MS [M+H] = 630 (M+1)

### Preparation Example 4: Preparation of amorphous compound of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

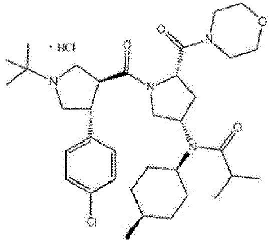

Based on 1 g of the compound (MC70) prepared in Preparation Example 3 above, 19 mL of MBTE was used to dissolve the compound (MC70) at 25°C. After dissolution was completed, 1 mL of heptane was added, and then the mixture was cooled to -5 to 0°C. After the set temperature was reached, 1 equivalent of 4 M HCl/EtOAc was added dropwise, the mixture was stirred for about 90 minutes, and filtered to obtain the title compound (MC71). (yield: about 90%)

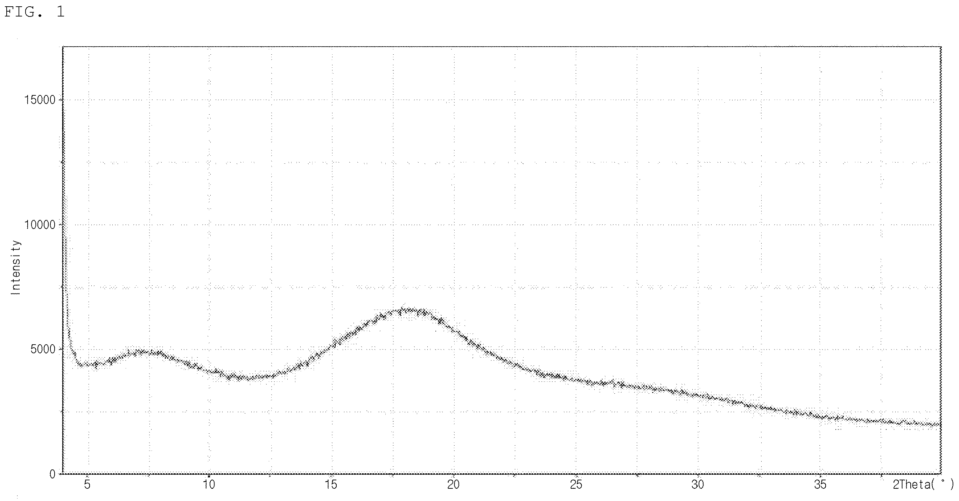

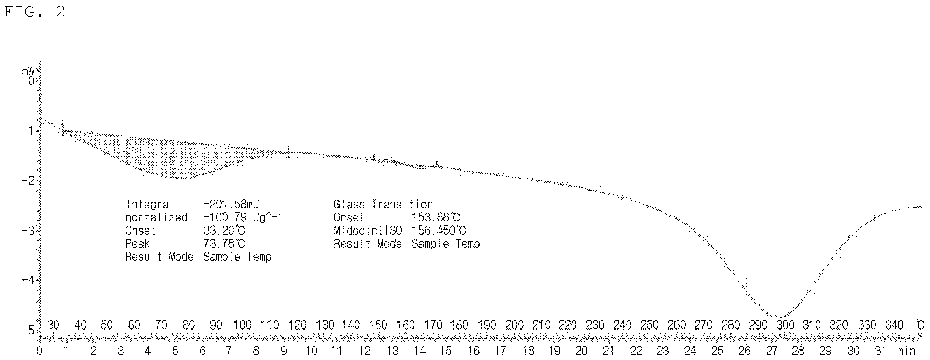

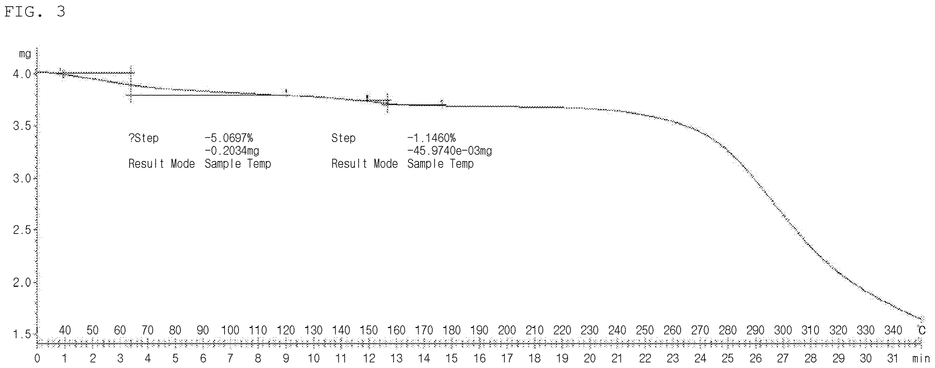

The XRD (FIG. 1), DSC (FIG. 2), and TGA (FIG. 3) analyses results for the compound of Preparation Example 4 were shown in FIGs. 1 to 3, respectively. The analyses results confirmed that it was an amorphous compound. The XRD, DSC, and TGA analysis methods each are as described in Experimental Examples for Example 1 below.

### Example 1.

### Preparation of crystalline form II of hydrate of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

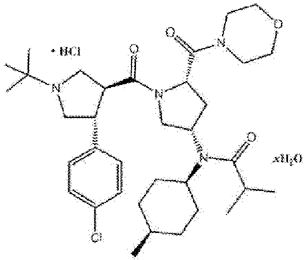

1 g of the compound (MC71) of Preparation Example 4 prepared above was dissolved in 0.6 mL of EtOAc and 0.06 mL of distilled water at room temperature while stirring with an electronic stirrer. After dissolution was completed, 0.6 mL of heptane was added dropwise, stirring was carried out 18 hours, and then filtration was carried out under nitrogen pressure to obtain the title crystalline form II. (yield: about 60%)

### Comparative Example 1

1 g of the compound (MC71) of Preparation Example 4 prepared above was dissolved in 1 mL of EtOAc at room temperature. After completion of dissolution, the mixture was stirred for about 43 hours with an electronic stirrer, but the same crystals as in Example 1 were not obtained.

### Experimental Example 1. XRD assessment

The powder XRD diffraction pattern was obtained using PANalytical X'Pert Pro MPD system equipped with a monochromatized radiation source and Ni filter as a solid-state detector by the following method.

About 20 to 30 mg of the sample was compressed in a glass sample holder so that the sample had a flat surface, the generator of the apparatus was set to 45 kV (acceleration voltage) and 40 mA (filament emission), and then, the measurement was carried out in a reflection mode (not-spin). Bragg angles (2θ) in a range of 4 to 40° were measured with the conditions of step size of 0.026° and time per step of 51 seconds.

The XRD measurement result of the obtained crystalline form II is shown in FIG. 4.

As can be seen from the spectrum shown in FIG. 4, the crystalline form II according to the present invention exhibited characteristic peaks (2θ) at 7.77°, 9.82°, 10.50°, 11.37°, 12.36°, 15.17°, 15.46°, 15.88°, 16.75°, 17.59°, 17.93°, 18.33°, 19.64°, 20.19°, 21.19°, 21.71°, 23.29°, 23.58°, 24.42°, 25.07°, 25.63°, 26.31°, 27.17°, 27.52°, and 28.96°. The specific values of the XRD are shown in Table 1 below.

**[Table 1]**

| 2θ | Relative Intensity (I/I₀) |
|---|---|
| 7.77 | 6541 |
| 9.82 | 7889 |
| 10.50 | 4522 |
| 11.37 | 5455 |
| 12, 36 | 5321 |
| 15.17 | 5416 |
| 15.46 | 5307 |
| 15.88 | 7127 |
| 16.75 | 841 9 |
| 17.59 | 4096 |
| 17.93 | 4131 |
| 18.33 | 3955 |
| 19.64 | 9251 |
| 20.19 | 12311 |
| 21.19 | 6879 |
| 21.71 | 6771 |
| 23.29 | 3855 |
| 23.58 | 4334 |
| 24.42 | 3725 |
| 25.07 | 3480 |
| 25.63 | 3732 |
| 26.31 | 3907 |
| 27.17 | 2856 |
| 27.52 | 2925 |
| 28.96 | 3443 |

### Experimental Example 2. Differential Scanning Calorimetry (DSC)

The DSC was measured using Mettler Toledo DSC1 system. 2-5 mg of the sample is weighed and put into a 40 µL Al crucible (flat-bottomed aluminum pan with one pin-hole lid), and one pinhole is made. Then, DSC measurement is performed while the sample is heated from 25°C to 350°C at a rate of 10°C/min. During the measurement, nitrogen gas is supplied to the inside of the instrument at a rate of 70 mL/min to prevent the inflow of oxygen and other gases. Data collection and evaluation were performed using the software STARe.

The DSC measurement result of the obtained crystalline form II is shown in FIG. 5.

As can be seen in FIG. 5, for the crystalline form II, the first endothermic peak was observed at about 28.8°C (onset), and the second endothermic peak was observed at about 172.6°C (onset). After about 220°C, an endothermic peak due to decomposition appeared. The temperature values have an error of ±5°C.

### Experimental Example 3. Thermogravimetric Analysis (TGA)

The TGA was measured using Mettler Toledo TGA/DSC 1 module. About 4-8 mg of the sample is weighed and put into a 100 µL Al crucible (flat-bottomed aluminum crucibles). Then, TGA measurement is performed while the sample is heated from 30°C to 350°C at a rate of 10°C/min. During the measurement, nitrogen gas is supplied to the inside of the instrument at a rate of 80 mL/min to prevent the inflow of oxygen and other gases. Data collection and evaluation were performed using the software STARe.

The TGA measurement result of the obtained crystalline form II is shown in FIG. 6.

As can be seen in FIG. 6, for the crystalline form II, a weight loss of about 5.9% was observed at a temperature of less than 170°C. After about 220°C, a weight loss due to decomposition occurred. The temperature values have an error of ±5°C. There was no weight loss in the endothermic section according to the DSC measurement result. Accordingly, it was confirmed that the crystalline form II was a thermally stable crystalline form.

### Experimental Example 4. Stability assessment

About 10-30 mg of the sample was stored for 4 weeks in an open state under the accelerated condition (40°C, 75% RH) or in a sealed state under the harsh condition in an oven at 80°C. To compare the samples with the samples stored at room temperature, HPLC analysis was performed by the method shown in Table 2 below.

**[Table 2]**

| | | |
|---|---|---|
| Column temperature | 30°C | |
| Mobile phase A | Acetonitrile/Trifluoroacetic acid = 100/0.1 (%, v/v) | |
| Mobile phase B | Water/Trifluoroacetic acid = 100/0.1 (%, v/v) | |

| Gradient system | | |
|---|---|---|
| Time (min) | Mobile phase A (%) | Mobile phase B (%) |
| 0 | 40 | 60 |
| 10 | 50 | 50 |
| 13 | 50 | 50 |
| 18 | 40 | 60 |
| Flow rate | 1 mL/min | |
| Detection wavelength | UV 214 nm | |
| Injection volume | 5µL | |

The results of assessing the stability of the obtained crystalline form II are shown in Table 3 below.

**[Table 3]**

| Time | Example 1 | |
|---|---|---|
| | Open ACC | Closed STR |
| Initial | 97.70 | |
| Week 2 | 91.57 | 97.73 |
| Week 4 | 97.82 | 97.94 |

As can be seen in Table 3 above, the crystalline form II according to the present invention showed chemical stability for 4 weeks under the accelerated condition (40°C, 75% RH) and the harsh condition (80°C). Thus, it was confirmed that the crystalline form II according to the present invention showed excellent stability to heat and humidity.

## Claims

1. A crystalline form II of a compound of the following formula 1, a pharmaceutically acceptable salt thereof, or a solvate thereof,
wherein the X-ray powder diffraction pattern has 3 or more characteristic peaks selected from among peaks with the following diffraction angles (2θ values) of: 7.77±0.2°, 9.8210.2°, 10.5010.2°, 11.3710.2°, 12.3610.2°, 15.1710.2°, 15.4610.2°, 15.8810.2°, 16.7510.2°, 17.5910.2°, 17.9310.2°, 18.3310.2°, 19.6410.2°, 20.1910.2°, 21.1910.2°, 21.7110.2°, 23.2910.2°, 23.5810.2°, 24.4210.2°, 25.07±0.2°, 25.6310.2°, 26.3110.2°, 27.1710.2°, 27.5210.2°, and 28.9610.2°: 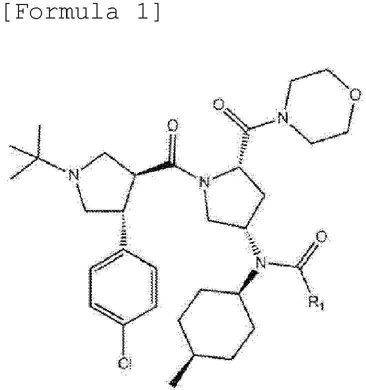
wherein R₁ is C₂-C₅ alkyl.

2. The crystalline form II of claim 1, wherein the pharmaceutically acceptable salt of the compound of formula 1 is selected from the group consisting of: hydrochloric acid, sulfuric acid, nitric acid, phosphoric acid, hydrobromic acid, and hydroiodic acid, of the compound.

3. The crystalline form II of claim 1, which is a crystalline form of a solvate of the compound of formula 1.

4. The crystalline form II of claim 3, wherein the solvate is a hydrate.

5. The crystalline form II of claim 4, which is a crystalline form of a compound of the following formula 4:

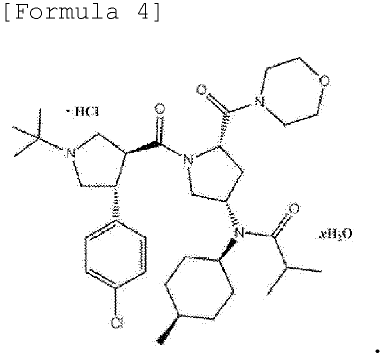

6. A method for preparing the crystalline form II described in any one of claims 1-5, the method comprising the steps of:
preparing a mixed solution by dissolving the compound of formula 1 in a crystallization solvent; and
obtaining crystals from the mixed solution.

7. The method for preparing the crystalline form II of claim 6, wherein the crystallization solvent includes water, a polar aprotic organic solvent, or a mixture thereof.

8. The method for preparing the crystalline form II of claim 7, wherein the polar aprotic organic solvent includes ethyl acetate, methyl isobutyl ketone, dimethyl sulfoxide, tetrahydrofuran, acetone, dimethylformamide, acetonitrile, or a mixture thereof.

9. The method for preparing the crystalline form II of claim 7, wherein the crystallization solvent is a mixed solvent in which water and the polar aprotic solvent are mixed in a volume ratio of 20:1 to 1:20.

10. The method for preparing the crystalline form II of claim 6, further comprising a step for adding a non-polar organic solvent to the mixed solution.

11. A pharmaceutical composition comprising the crystalline form II according to any one of claims 1-5 and a pharmaceutically acceptable carrier.

12. A pharmaceutical composition for agonizing the function of a melanocortin-4 receptor, the composition comprising the crystalline form II according to any one of claims 1-5 and a pharmaceutically acceptable carrier.

13. The pharmaceutical composition of claim 12, which is for preventing or treating obesity, diabetes, inflammation, or erectile dysfunction.
